# DEMANDE DE BREVET EUROPEEN

(11) **EP 3 599 275 A1**
(43) Date de publication de la demande: **29.01.2020**
(21) Numéro de dépôt: 18306016.9
(22) Date de dépôt: 27.07.2018
(51) Int. Cl.: C12M 1/12, A61L 2/16, C12M 1/34

(54) **INSTALLATION DE TRAITEMENT DE LIQUIDE BIOLOGIQUE**

(71) Demandeur: EMD Millipore Corporation, Burlington, MA 01803 (US)
(72) Inventeur: JOURDAINNE, Laurent, BURLINGTON, MA 01803, (US); CIROU, Sébastien, BURLINGTON, MA 01803, (US)
(74) Mandataire: Santarelli

(57) **Abrégé**

L'invention concerne une installation de traitement de liquide biologique par inactivation virale, comportant une vanne principale d'alimentation (4) en liquide biologique à traiter, un premier circuit (2) en aval de ladite vanne et pourvu d'un premier réservoir principal (7), un deuxième circuit (3) en aval de ladite vanne et pourvu d'un deuxième réservoir principal (14), ledit deuxième circuit étant parallèle audit premier circuit, et un troisième réservoir principal (11) disposé en sortie à la fois dudit premier circuit et dudit deuxième circuit et configuré pour être alimenté successivement par ledit premier réservoir principal et par ledit deuxième réservoir principal ; ladite installation étant configurée pour que dans chacun desdits premier, deuxième et troisième réservoirs principaux, un volume déterminé d'acide et un volume déterminé de base est introduit de sorte au moins à ajuster un pH dudit liquide biologique et pour réguler un débit de vidange dudit troisième réservoir principal de sorte à fournir un débit continu de liquide biologique traité en sortie de ladite installation.

## Description

### DOMAINE DE L'INVENTION

L'invention a trait aux installations de traitement de liquide biologique, en particulier mais non exclusivement pour l'inactivation virale d'un liquide biopharmaceutique.

### ARRIERE PLAN TECHNOLOGIQUE

Les liquides biopharmaceutiques sont en général obtenus par cultures en bioréacteur et il faut ensuite les purifier pour atteindre les caractéristiques requises de pureté, de concentration, d'absence de virus, etc.

La purification peut être réalisée au moyen d'une succession de traitements tels que la clarification pour éliminer les résidus de la culture en bioréacteur, l'élution par chromatographie pour séparer des protéines, l'inactivation virale puis le polissage et/ou la purification par filtration tangentielle ou par chromatographie.

Il est souhaitable de réaliser les étapes ci-dessus les unes après les autres et de manière continue ou au moins semi-continue. Cependant, ces étapes de traitement ne prennent pas toutes le même temps et il est parfois nécessaire d'arrêter certains traitements le temps qu'un ou plusieurs autres traitements soient terminés. En particulier, on sait que le temps de traitement pour l'inactivation virale requière généralement un temps d'incubation pendant lequel le flux de produit est stoppé.

La demande de brevet EP 2 682 168 a trait à un traitement d'inactivation virale et décrit un procédé où un flux de produit est fractionné en petits volumes de produit, aussi appelés fractions. Ce procédé permet une désactivation virale particulièrement rapide de chaque petite fraction (ou volume) de produit les unes après les autres.

On connait également une installation d'inactivation virale, comportant un distributeur central alimenté en produit à traiter, en acide et en base, et configuré pour aiguiller différents flux de produit, d'acide et de base, dont deux flux dits de traitement vers deux réservoirs respectifs, et un flux dit de sortie débouchant soit sur un drain soit sur une conduite de sortie du produit traité. L'installation est en outre pourvue de deux boucles de recirculations des deux flux de traitement prévues chacune entre un réservoir respectif et le distributeur central.

### OBJET DE L'INVENTION

L'invention vise à fournir une installation permettant la mise en oeuvre d'un traitement d'inactivation virale d'un liquide biologique de manière simple, commode et économique.

Pour cela, l'invention concerne, sous un premier aspect, une installation de traitement de liquide biologique par inactivation virale, caractérisée en ce qu'elle comporte :
une vanne principale d'alimentation en liquide biologique à traiter, configurée pour être raccordée au moins à une source d'alimentation en liquide biologique ;
un premier circuit de traitement en aval de ladite vanne d'alimentation et pourvu d'un premier réservoir principal de traitement configuré pour être alimenté par ladite vanne d'alimentation en liquide biologique à traiter ;
un deuxième circuit de traitement en aval de ladite vanne d'alimentation et pourvu d'un deuxième réservoir principal de traitement configuré pour être alimenté par ladite vanne d'alimentation en liquide biologique à traiter ;
ledit deuxième circuit de traitement étant parallèle audit premier circuit de traitement ; et
un troisième réservoir principal de traitement disposé en sortie à la fois dudit premier circuit de traitement et dudit deuxième circuit de traitement et configuré pour être alimenté successivement par ledit premier réservoir principal de traitement et par ledit deuxième réservoir principal de traitement ;
ladite installation est configurée pour que dans chacun desdits premier, deuxième et troisième réservoirs principaux de traitement, un volume déterminé d'acide et un volume déterminé de base est introduit de sorte au moins à ajuster un pH dudit liquide biologique ; et ladite installation est aussi configurée pour réguler un débit de vidange dudit troisième réservoir principal de traitement de sorte à fournir un débit continu de liquide biologique traité en sortie de ladite installation.

En d'autres termes, dans l'installation de traitement de liquide biologique selon l'invention, le troisième réservoir principal de traitement est commun aux premier et deuxième circuits de traitement.

Les premier et deuxième circuits de traitement s'étendent donc parallèlement depuis la vanne principale d'alimentation jusqu'au troisième réservoir principal de traitement.

Au surplus, dans l'installation de traitement de liquide biologique selon l'invention, le troisième réservoir principal de traitement permet d'achever le traitement du liquide biologique déjà traité dans le premier réservoir principal de traitement ou dans le deuxième réservoir principal de traitement. En particulier, le troisième réservoir principal de traitement peut être prévu pour pré-polir le liquide biologique, c'est-à-dire pour que ce dernier retrouve un pH cible. Une fois le troisième réservoir principal de traitement rempli de liquide biologique traité et une fois le pré-polissage effectué, le troisième réservoir principal de traitement se vide avec un débit contrôlé et continu.

A noter qu'un tel pré-polissage permet de préparer le liquide biologique pour une étape postérieure de polissage de ce liquide, par exemple à l'aide d'un traitement par chromatographie.

Selon d'autres caractéristiques préférées, simples, commodes et économiques de l'installation selon l'invention :
- ledit premier circuit de traitement est pourvu d'un premier réservoir intermédiaire de traitement disposé entre lesdits premier et troisième réservoirs principaux de traitement ;
- ladite installation est configurée pour qu'un volume déterminé d'acide et qu'un volume déterminé de base soient introduits dans ledit premier réservoir intermédiaire de traitement ;
- ladite installation est configurée pour que ledit liquide biologique traité dans ledit premier réservoir principal de traitement soit transféré dans ledit premier réservoir intermédiaire de traitement et incube un temps prédéterminé dans ce dernier, avant son transfert dans ledit troisième réservoir principal de traitement ;

- ledit deuxième circuit de traitement est pourvu d'un deuxième réservoir intermédiaire de traitement disposé entre lesdits deuxième et troisième réservoirs principaux de traitement ;
- ladite installation est configurée pour qu'un volume déterminé d'acide et qu'un volume déterminé de base soient introduits dans ledit deuxième réservoir intermédiaire de traitement ;
- ladite installation est configurée pour que ledit liquide biologique traité dans ledit deuxième réservoir principal de traitement soit transféré dans ledit deuxième réservoir intermédiaire de traitement et incube un temps prédéterminé dans ce dernier, avant son transfert dans ledit troisième réservoir principal de traitement ;
- ladite installation comporte une pluralité de pompes d'alimentation disposées sur lesdits premiers et deuxième circuits de traitement, au moins entre ledit premier réservoir principal de traitement et ledit troisième réservoir principal de traitement, voire entre ledit premier réservoir principal de traitement et ledit premier réservoir intermédiaire de traitement et/ou entre ledit premier réservoir intermédiaire de traitement et ledit troisième réservoir principal de traitement ; et au moins entre ledit deuxième réservoir principal de traitement et ledit troisième réservoir principal de traitement, voire entre ledit deuxième réservoir principal de traitement et ledit deuxième réservoir intermédiaire de traitement et/ou entre ledit deuxième réservoir intermédiaire de traitement et ledit troisième réservoir principal de traitement ;
- lesdits volumes déterminés d'acide, respectivement lesdits volumes déterminés de base, introduits au moins dans lesdits premier, deuxième et troisième réservoirs principaux de traitement, voire dans lesdits premier et deuxième réservoirs intermédiaires de traitement, dépendent des propriétés physicochimiques du liquide biologique à traiter et peuvent être égaux ou différents ;
- au moins lesdits premier, deuxième et troisième réservoirs principaux de traitement, voire lesdits premier et deuxième réservoirs intermédiaires de traitement, sont pourvus d'un agitateur de liquide biologique et/ou d'un ou plusieurs organes instrumentation ;
- ladite installation comporte une ou plusieurs unités de contrôle et de commande configurées pour contrôler et commander ladite vanne d'alimentation et/ou au moins un desdits premier, deuxième et troisième réservoirs principaux de traitement, voire pour contrôler et commander lesdits premier et deuxième réservoirs intermédiaires de traitement ;
- ladite installation comporte un unique chariot sur lequel sont disposés au moins ladite vanne d'alimentation, lesdits premier, deuxième et troisième réservoirs principaux de traitement, voire lesdits premier et deuxième réservoirs intermédiaires de traitement ; et/ou
- ladite installation est pourvue de conduites jetables, dites à usage unique, reliant différents éléments la composant, dont au moins ladite vanne d'alimentation, lesdits premier, deuxième et troisième réservoirs principaux de traitement, voire lesdits premier et deuxième réservoirs intermédiaires de traitement.

L'invention concerne aussi, sous un second aspect, un procédé de traitement de liquide biologique par inactivation virale, à l'aide d'une installation de traitement telle que décrite ci-dessus, ledit procédé comportant :
une étape de remplissage au moins partiel en liquide biologique à traiter d'un premier réservoir principal de traitement d'un premier circuit de traitement de ladite installation via une vanne principale d'alimentation de ladite installation ;
une étape d'ajustement de pH dudit liquide biologique en introduisant un volume déterminé d'acide et un volume déterminé de base dans ledit premier réservoir principal de traitement ;
une étape d'incubation dudit liquide biologique dans ledit premier circuit de traitement pendant un temps prédéterminé et une étape d'ajustement de pH dudit liquide biologique en introduisant un volume déterminé d'acide et un volume déterminé de base dans ledit premier circuit de traitement pour se rapprocher d'un pH cible ;
une étape de transfert dudit liquide biologie traité dans un troisième réservoir principal de traitement de ladite installation disposé en sortie dudit premier circuit de traitement, et une étape de pré-polissage en introduisant un volume déterminé d'acide et un volume déterminé de base dans ledit troisième réservoir principal de traitement pour obtenir un liquide biologique traité au pH cible ;
en même que les étapes de pré-polissage et/ou de transfert et/ou d'incubation et/ou d'ajustements de pH mises en oeuvre dans ledit premier circuit de traitement, une étape de remplissage au moins partiel en liquide biologique à traiter d'un deuxième réservoir principal de traitement d'un deuxième circuit de traitement de ladite installation via ladite vanne principale d'alimentation, ledit deuxième circuit de traitement étant parallèle audit premier circuit de traitement ;
une étape d'ajustement de pH dudit liquide biologique en introduisant un volume déterminé d'acide et un volume déterminé de base dans ledit deuxième réservoir principal de traitement ;
une étape d'incubation dudit liquide biologique dans ledit deuxième circuit de traitement pendant un temps prédéterminé et une étape d'ajustement de pH dudit liquide biologique en introduisant un volume déterminé d'acide et un volume déterminé de base dans ledit deuxième circuit de traitement pour se rapprocher d'un pH cible ;
une étape de transfert dudit liquide biologie traité dans ledit troisième réservoir principal de traitement disposé aussi en sortie dudit deuxième circuit de traitement, et une étape de pré-polissage en introduisant un volume déterminé d'acide et un volume déterminé de base dans ledit troisième réservoir principal de traitement pour obtenir un liquide biologique traité au pH cible ;
en même que les étapes de pré-polissage et/ou de transfert et/ou d'incubation et/ou d'ajustements de pH mises en oeuvre dans ledit deuxième circuit de traitement, une étape de vidange dudit troisième réservoir principal de traitement en régulant un débit de vidange de sorte à fournir un débit continu de liquide biologique traité en sortie de ladite installation.

Selon d'autres caractéristiques préférées, simples, commodes et économiques du procédé de traitement selon l'invention, ledit premier circuit de traitement est pourvu d'un premier réservoir intermédiaire de traitement disposé entre lesdits premier et troisième réservoirs principaux de traitement et ledit deuxième circuit de traitement est pourvu d'un deuxième réservoir intermédiaire de traitement disposé entre lesdits deuxième et troisième réservoirs principaux de traitement ; et le procédé comporte une étape de transfert dudit liquide biologique depuis ledit premier réservoir principal de traitement vers ledit premier réservoir intermédiaire de traitement et les étapes d'incubation et d'ajustement de pH pour s'approcher d'un pH cible dans ledit premier circuit de traitement ont lieu dans ledit premier réservoir intermédiaire de traitement ; ainsi qu'une étape de transfert dudit liquide biologique depuis ledit deuxième réservoir principal de traitement vers ledit deuxième réservoir intermédiaire de traitement et les étapes d'incubation et d'ajustement de pH pour s'approcher d'un pH cible dans ledit deuxième circuit de traitement ont lieu dans ledit deuxième réservoir intermédiaire de traitement.

### BREVE DESCRIPTION DES DESSINS

L'exposé de l'invention sera maintenant poursuivi par la description d'un exemple de réalisation, donnée ci-après à titre illustratif et non limitatif, en référence aux dessins annexés sur lesquels :
- la figure 1 représente schématiquement une installation de traitement conforme à l'invention ;
- la figure 2 est un chronogramme montrant différentes étapes d'un procédé de traitement utilisant l'installation de la figure 1 ; et
- les figures 3 à 5 sont des vues en perspective et de dessus de l'installation de traitement de la figure 1.

### DESCRIPTION DETAILLEE D'AU MOINS UN EXEMPLE DE REALISATION

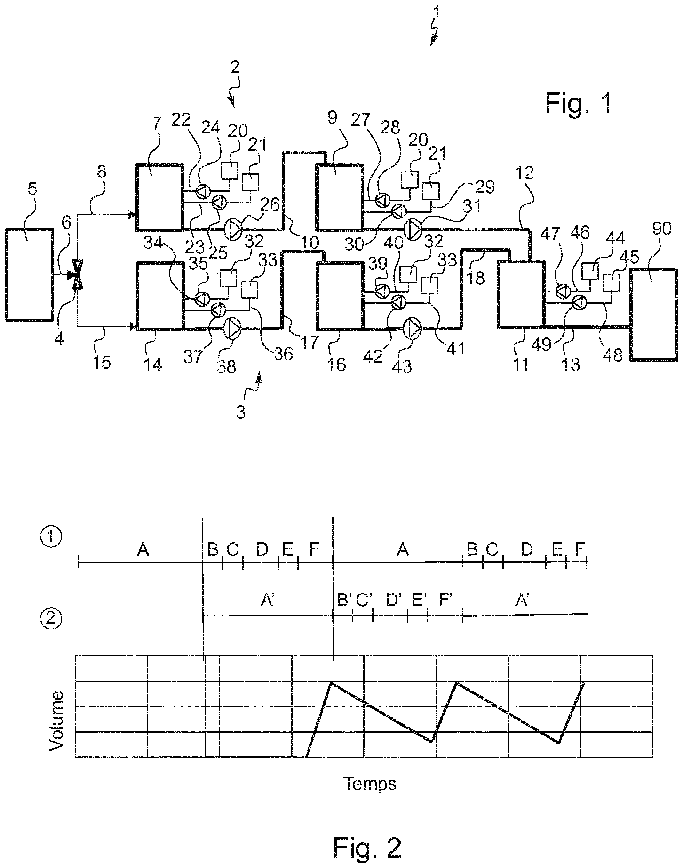

La figure 1 illustre schématiquement une installation de traitement par inactivation de liquide biologique 1.

L'installation comporte un premier circuit de traitement 2 et un deuxième circuit de traitement 3 qui s'étend parallèlement au premier circuit de traitement 2.

L'installation 1 peut comporter en outre, en amont des premier et deuxième circuits de traitement 2 et 3, une source d'alimentation en liquide biologique 5 et une vanne principale d'alimentation en liquide biologique à traiter 4 raccordée à la source d'alimentation en liquide biologique 5 via une première conduite 6.

A noter que cette source d'alimentation peut être directement une conduite de sortie d'un circuit de traitement d'une installation amont à l'installation 1 ; ou bien un récipient individuel connecté ou non à une telle installation amont.

Le premier circuit de traitement 2 peut être pourvu d'un premier réservoir principal de traitement 7 configuré pour être alimenté en liquide biologique à traiter par la vanne d'alimentation 4 via une deuxième conduite 8, et d'un premier réservoir intermédiaire de traitement 9 disposé en aval du premier réservoir principal de traitement 7 et raccordé à ce dernier via une troisième conduite 10.

Le deuxième circuit de traitement 3 peut être pourvu d'un deuxième réservoir principal de traitement 14 configuré pour être alimenté en liquide biologique à traiter par la vanne d'alimentation 4 via une quatrième conduite 15, et d'un deuxième réservoir intermédiaire de traitement 16 disposé en aval du deuxième réservoir principal de traitement 14 et raccordé à ce dernier via une cinquième conduite 17.

L'installation 1 peut comporter également, en aval des premier et deuxième circuits de traitement 2 et 3, un circuit de sortie formé principalement ici par un troisième réservoir principal de traitement 11 qui est raccordé au premier réservoir intermédiaire de traitement 9 via une sixième conduite 12, et qui est aussi raccordé au deuxième réservoir intermédiaire de traitement 16 via une septième conduite 18.

Ainsi, le premier réservoir intermédiaire de traitement 9 est interposé entre le premier réservoir principal de traitement 7 et le troisième réservoir principal de traitement 11 ; tandis que le deuxième réservoir intermédiaire de traitement 16 est interposé entre le deuxième réservoir principal de traitement 14 et le troisième réservoir principal de traitement 11.

Le troisième réservoir principal de traitement 11 est disposé en sortie à la fois des premier et deuxième circuits de traitement 2 et 3 et configuré pour être alimenté successivement par le premier réservoir principal de traitement 7 et par le deuxième réservoir intermédiaire de traitement 16.

En d'autres termes, le troisième réservoir principal de traitement 11 est commun aux premier et deuxième circuits de traitement 2 et 3.

Les premier et deuxième circuits de traitement 2 et 3 s'étendent parallèlement depuis la vanne principale d'alimentation 4 jusqu'au troisième réservoir principal de traitement 11.

L'installation 1 peut être configurée pour que le liquide biologique traité dans le premier réservoir principal de traitement 7, respectivement dans le deuxième réservoir principal de traitement 14, soit transféré dans le premier réservoir intermédiaire de traitement 9, respectivement dans le deuxième réservoir intermédiaire de traitement 16, et incube un temps prédéterminé dans ce dernier, avant son transfert dans le troisième réservoir principal de traitement 11.

L'installation 1 peut comporter pour cela une première pompe principale d'alimentation 26 montée sur la troisième conduite 10 et configurée pour vidanger le premier réservoir principal de traitement 7 et transférer le liquide biologique traité dans ce dernier vers le premier réservoir intermédiaire de traitement 9 ; ainsi qu'une première pompe intermédiaire d'alimentation 31 montée sur la sixième conduite 12 et configurée pour vidanger le premier réservoir intermédiaire de traitement 9 et transférer le liquide biologique traité dans ce dernier vers le troisième réservoir principal de traitement 11.

L'installation 1 peut comporter en outre une deuxième pompe principale d'alimentation 38 montée sur la cinquième conduite 17 et configurée pour vidanger le deuxième réservoir principal de traitement 14 et transférer le liquide biologique traité dans ce dernier vers le deuxième réservoir intermédiaire de traitement 16 ; ainsi qu'une deuxième pompe intermédiaire d'alimentation 43 montée sur la septième conduite 18 et configurée pour vidanger le deuxième réservoir intermédiaire de traitement 16 et transférer le liquide biologique traité dans ce dernier vers le troisième réservoir principal de traitement 11.

L'installation 1 peut aussi être configurée pour que dans chacun des premier, deuxième et troisième réservoirs principaux de traitement 7, 14 et 11 et des premier et deuxième réservoirs intermédiaires de traitement 9 et 16, un volume déterminé d'acide et un volume déterminé de base est introduit de sorte au moins à ajuster un pH du liquide biologique.

L'installation 1 peut comporter pour cela un premier réservoir d'un acide 20 qui est raccordé au premier réservoir principal de traitement 7 via une huitième conduite 22 sur laquelle est montée une première pompe acide 24, et qui est aussi raccordé au premier réservoir intermédiaire de traitement 9 via une neuvième conduite 27 sur laquelle est montée une deuxième pompe acide 28.

L'installation 1 peut comporter également un premier réservoir d'une base 21 qui est raccordé au premier réservoir principal de traitement 7 via une dixième conduite 23 sur laquelle est montée une première pompe base 25, et qui est aussi raccordé au premier réservoir intermédiaire de traitement 9 via une onzième conduite 29 sur laquelle est montée une deuxième pompe base 30.

L'installation 1 peut comporter en outre un deuxième réservoir d'un acide 32 qui est raccordé au deuxième réservoir principal de traitement 14 via une douzième conduite 34 sur laquelle est montée une troisième pompe acide 35, et qui est aussi raccordé au deuxième réservoir intermédiaire de traitement 16 via une treizième conduite 40 sur laquelle est montée une quatrième pompe acide 39.

L'installation 1 peut comporter au surplus un deuxième réservoir d'une base 32 qui est raccordé au deuxième réservoir principal de traitement 14 via une quinzième conduite 36 sur laquelle est montée une troisième pompe base 37, et qui est aussi raccordé au deuxième réservoir intermédiaire de traitement 16 via une seizième conduite 41 sur laquelle est montée une quatrième pompe base 42.

L'installation 1 peut comporter aussi un troisième réservoir d'un acide 44 qui est raccordé au troisième réservoir principal de traitement 11 via une dix-septième conduite 46 sur laquelle est montée une cinquième pompe acide 47, et un troisième réservoir de base 45 qui est raccordé au troisième réservoir principal de traitement 11 via une dix-huitième conduite 48 sur laquelle est montée une cinquième pompe base 49.

On notera que le troisième réservoir principal de traitement 11 peut être vidangé par le biais d'une conduite de sortie 13, laquelle est raccordée à un circuit 90 (représenté très schématiquement) dans une installation disposée en aval de l'installation 1. Ce circuit 90 peut être configuré pour mettre en oeuvre une étape de polissage du liquide biologique, par exemple par chromatographie.

Toutes les conduites 6, 8, 10, 12, 13, 15, 17, 18, 22, 23, 27, 29, 34, 36, 40, 41, 46 et 48 peuvent par exemple être des conduites souples jetables, dites à usage unique.

On notera également que l'installation 1 est ici configurée pour réguler au moins un débit de vidange du troisième réservoir principal de traitement 11 de sorte à fournir un débit continu de liquide biologique traité en sortie de la installation 1 (voir ci-après). La valeur du débit est fonction du circuit 90 en amont de l'installation 1 et du traitement mis en oeuvre, ici un polissage du liquide biologique.

On va maintenant décrire, en référence à la figure 2, un procédé de traitement de liquide biologique par inactivation virale, à l'aide de l'installation de traitement 1 décrite ci-dessus.

En particulier, la figure 2 représente, à la façon d'un chronogramme, sur sa première ligne 1, différentes étapes mises en oeuvre sur le premier circuit de traitement 2, sur sa deuxième ligne 2, différentes étapes mises en oeuvre sur le deuxième circuit de traitement 3, et sur sa troisième ligne, le volume de liquide biologique dans le troisième réservoir principal de traitement 11.

Le procédé de traitement de liquide biologique par inactivation virale, à l'aide de l'installation de traitement 1 comporte les étapes ci-dessous.

Le procédé peut comporter une étape de remplissage A au moins partiel en liquide biologique à traiter du premier réservoir principal de traitement 7 via la vanne principale d'alimentation 4 et la deuxième conduite 8.

Le temps de remplissage est fonction du volume du premier réservoir principal de traitement 7.

Le procédé peut comporter une étape d'ajustement de pH B du liquide biologique présent dans le premier réservoir principal de traitement 7 en introduisant un volume déterminé d'acide et un volume déterminé de base dans ce premier réservoir principal de traitement 7, via la huitième conduite 22 et la première pompe acide 24 et via la dixième conduite 23 et la première pompe base 25.

Les volumes d'acide et de base dépendent de la valeur de pH du liquide biologique en entrée dans le premier réservoir principal de traitement 7.

Le procédé peut comporter une étape d'incubation du liquide biologique dans le premier circuit de traitement 2 pendant un temps prédéterminé et une étape d'ajustement de pH du liquide biologique en introduisant un volume déterminé d'acide et un volume déterminé de base dans le premier circuit de traitement 2 pour se rapprocher d'un pH cible.

A noter que le pH cible peut correspondre à un pH neutre ou bien être différent d'un pH neutre. La valeur du pH cible dépend du type de traitement mis en oeuvre dans le circuit 90 dans l'installation qui succède l'installation 1.

En particulier, le procédé peut comporter une étape de transfert C du liquide biologique depuis le premier réservoir principal de traitement 7 vers le premier réservoir intermédiaire de traitement 9 via la première pompe principale d'alimentation 26 et la troisième conduite 10 ; une étape d'incubation D du liquide biologique dans le premier réservoir intermédiaire de traitement 9 ; et une étape d'ajustement de pH E pour s'approcher d'un pH cible, en introduisant un volume déterminé d'acide et un volume déterminé de base dans ce premier réservoir intermédiaire de traitement 9, via la neuvième conduite 27 et la deuxième pompe acide 28 et via la onzième conduite 29 et la deuxième pompe base 30.

Le procédé peut comporter une étape de transfert F du liquide biologie traité depuis le premier réservoir intermédiaire de traitement 9 vers le troisième réservoir principal de traitement 11 via la première pompe intermédiaire d'alimentation 31 et la sixième conduite 12.

En même temps que l'étape de transfert F, et sous réserve d'avoir transféré un volume suffisant déterminé, le procédé peut comporter une étape de pré-polissage du liquide biologique dans le troisième réservoir principal de traitement 11, en introduisant un volume déterminé d'acide et un volume déterminé de base dans le troisième réservoir principal de traitement 11, via la dix-septième conduite 46 et la cinquième pompe acide 47 et via la dix-huitième conduite 48 et la cinquième pompe base 49 ; pour obtenir un liquide biologique traité au pH cible.

Les étapes A à F décrites ci-dessus concernent le premier circuit de traitement 2. Parallèlement aux étapes B à F, c'est-à-dire après remplissage du premier réservoir principal de traitement 7, le procédé peut comporter en même temps les étapes suivantes.

Le procédé peut comporter une étape de remplissage A' au moins partiel en liquide biologique à traiter du deuxième réservoir principal de traitement 14 via la vanne principale d'alimentation 4 et la quatrième conduite 15.

Le temps de remplissage est fonction du volume du deuxième réservoir principal de traitement 14. Ici les volumes des premier et deuxième réservoirs principaux de traitement 7 et 14 sont identiques.

Le procédé peut comporter une étape d'ajustement de pH B' du liquide biologique présent dans le deuxième réservoir principal de traitement 14 en introduisant un volume déterminé d'acide et un volume déterminé de base dans ce deuxième réservoir principal de traitement 14, via la douzième conduite 34 et la troisième pompe acide 35 et via la quinzième conduite 36 et la troisième pompe base 37.

Les volumes d'acide et de base dépendent de la valeur de pH du liquide biologique en entrée dans le deuxième réservoir principal de traitement 14.

Le procédé peut comporter une étape d'incubation du liquide biologique dans le deuxième circuit de traitement 3 pendant un temps prédéterminé et une étape d'ajustement de pH du liquide biologique en introduisant un volume déterminé d'acide et un volume déterminé de base dans le deuxième circuit de traitement 3 pour se rapprocher d'un pH cible.

En particulier, le procédé peut comporter une étape de transfert C' du liquide biologique depuis le deuxième réservoir principal de traitement 14 vers le deuxième réservoir intermédiaire de traitement 16 via la deuxième pompe principale d'alimentation 38 et la cinquième conduite 17 ; et une étape d'incubation D' du liquide biologique dans le deuxième réservoir intermédiaire de traitement 16 ; et une étape d'ajustement de pH E' pour s'approcher d'un pH cible, en introduisant un volume déterminé d'acide et un volume déterminé de base dans ce deuxième réservoir intermédiaire de traitement 16, via la treizième conduite 40 et la quatrième pompe acide 39 et via la seizième conduite 41 et la quatrième pompe base 42.

Le procédé peut comporter une étape de transfert F' du liquide biologie traité depuis le deuxième réservoir intermédiaire de traitement 16 vers le troisième réservoir principal de traitement 11 via la deuxième pompe intermédiaire d'alimentation 43 et la septième conduite 18.

En même temps que l'étape de transfert F', et sous réserve également 'avoir transféré un volume suffisant déterminé, le procédé peut comporter une étape de pré-polissage du liquide biologique dans le troisième réservoir principal de traitement 11, en introduisant un volume déterminé d'acide et un volume déterminé de base dans le troisième réservoir principal de traitement 11, via la dix-septième conduite 46 et la cinquième pompe acide 47 et via la dix-huitième conduite 48 et la cinquième pompe base 49 ; pour obtenir un liquide biologique traité au pH cible.

Les étapes A' à F' décrites ci-dessus concernent le deuxième circuit de traitement 3. Parallèlement aux étapes B' à F', c'est-à-dire après remplissage du deuxième réservoir principal de traitement 14, le procédé peut mettre en oeuvre à nouveau l'étape A de remplissage du premier réservoir principal de traitement 7 puis les étapes B à F et ainsi de suite, comme cela est visible sur la figure 2.

La figure 2 montre également que le procédé peut comporter l'étape de vidanger le troisième réservoir principal de traitement 11 lorsque que ce dernier a été rempli par le premier circuit de traitement 2 et que le liquide biologique y a été pré-poli.

La vidange du troisième réservoir principal de traitement 11 se fait à un débit régulé et peut durer environ le temps des étapes B' à E' mises en oeuvre dans le deuxième circuit de traitement 3, après lesquelles le troisième réservoir principal de traitement 11 peut à nouveau rempli mais par le deuxième circuit de traitement 3, et ainsi de suite. On notera que le troisième réservoir principal de traitement 11 peut ne jamais être vidé complètement dès lors qu'il a été rempli.

La régulation du débit de sortie du troisième réservoir principal de traitement 11 permet de fournir un débit continu de liquide biologique traité en sortie de l'installation 1.

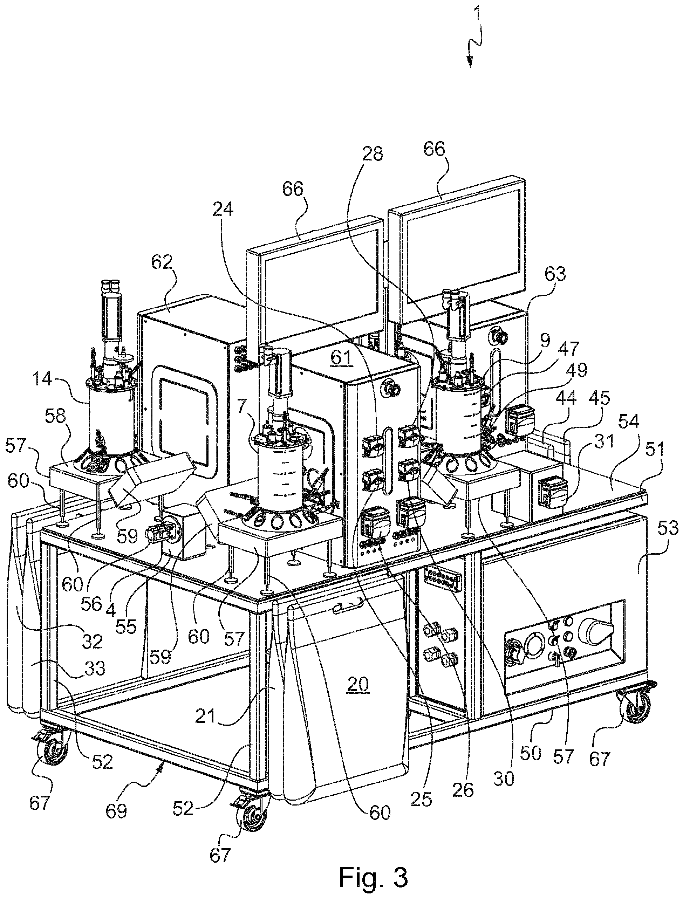

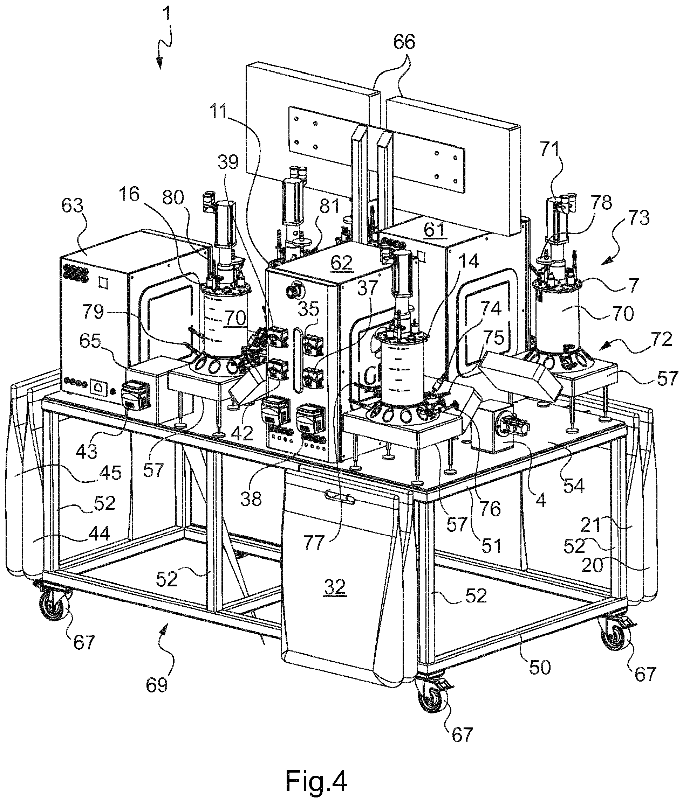

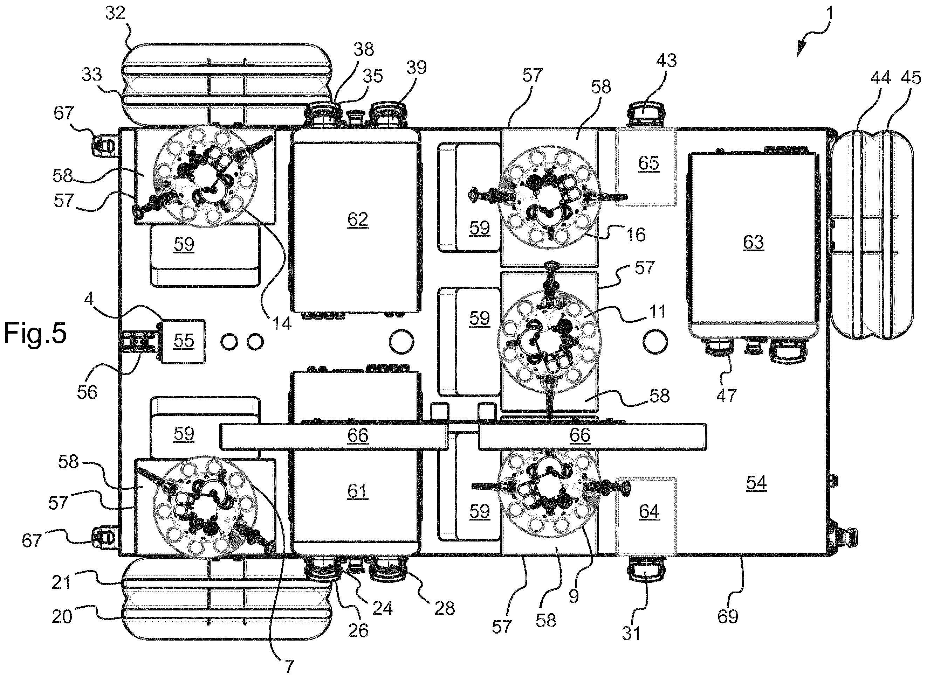

On va maintenant décrire plus en détail, en référence aux figures 3 à 5, l'agencement de l'installation 1 proprement dite et de ses composants, portés dans le mode de réalisation illustré ici par un unique chariot 69.

Le chariot 69 peut être formé par une structure en cadre, dont un cadre inférieur 50 monté sur des roulettes 67, un cadre supérieur 51 à distance du cadre inférieur 50, et des montants verticaux 52 s'étendant entre le cadre inférieur 50 et le cadre supérieur 51, et par un plateau 54 assujetti sur le cadre supérieur 51.

L'installation 1 peut comporter une unité générale d'alimentation électrique et pneumatique 53 posé sur le cadre inférieur 50.

Le chariot 69 peut avoir une forme globalement parallélépipédique.

Les premiers réservoirs d'acide 20 et de base 21 peuvent être suspendus, au niveau du cadre supérieur 51, sur un premier côté du chariot 69.

Les deuxièmes réservoirs d'acide 32 et de base 33 peuvent être suspendus, au niveau du cadre supérieur 51, sur un deuxième côté du chariot 69 opposé au premier côté.

Les troisièmes réservoirs d'acide 44 et de base 45 peuvent être suspendus, au niveau du cadre supérieur 51, sur un troisième côté du chariot 69 reliant ses premier et deuxième côtés, en sortie de l'installation 1.

Tous ces réservoirs peuvent être formés par des poches souples. A noter que la source d'alimentation n'est pas visible sur les figures 3 à 5.

La vanne d'alimentation 4 peut être posée sur le plateau 54 au niveau d'un quatrième côté du chariot 69, reliant aussi ses premier et deuxième côtés et étant opposé au troisième côté.

La vanne d'alimentation 4 peut être une vanne trois voies, dont deux entrées et une sortie ou une entrée et deux sorties.

La vanne 4 peut présenter par exemple une tête 56 par exemple pourvue de deux gorges de réception de portions de conduites et d'un mécanisme à pincement configuré pour autoriser ou interdire la circulation du liquide biologique dans les portions de conduites reçues dans les deux gorges ; et un corps 55 pourvu d'un actionneur pneumatique configuré pour actionner le mécanisme à pincement.

Les premier et deuxième réservoirs principaux de traitement 7 et 14 peuvent être disposés sur le plateau 54 au niveau du quatrième côté du chariot 69 et de part et d'autre de la vanne d'alimentation 4.

Ainsi, le premier réservoir principal de traitement 7 peut se situer sur le premier côté du chariot 69 tandis que le deuxième réservoir principal de traitement 14 se situe sur le deuxième côté du chariot 69.

Les premier et deuxième réservoirs principaux de traitement 7 et 14 peuvent être tous deux montés sur des balances respectives 57, pourvues chacune de pieds 60 sur lesquels est installé un plateau de balance 58 et d'un dispositif de contrôle et de commande de la balance 59.

Les premier et deuxième réservoirs principaux de traitement 7 et 14 peuvent être directement montés sur les plateaux de balance 58.

L'installation 1 peut comporter une première unité de contrôle et de commande 61 et une deuxième unité de contrôle et de commande 62 qui peuvent être disposées sur le plateau 54 à proximité respectivement des premier et deuxième réservoirs principaux de traitement 7 et 14.

La première unité de contrôle et de commande 61 peut être pourvue d'un corps duquel saille partiellement la première pompe acide 24, la première pompe base 25, la première pompe principale d'alimentation 26, la deuxième pompe acide 28 et la deuxième pompe base 30, du premier côté du chariot 69.

La deuxième unité de contrôle et de commande 62 peut être pourvue d'un corps duquel saille partiellement la troisième pompe acide 35, la troisième pompe base 37, la deuxième pompe principale d'alimentation 38, la quatrième pompe acide 39 et la quatrième pompe base 42, du deuxième côté du chariot 69.

Les première et deuxième unités de contrôle et de commande 61 et 62 peuvent ainsi être configurées pour contrôler et commander les pompes susmentionnées pour mettre en oeuvre au moins les étapes d'ajustement de pH B et B' au sein des premier et deuxième réservoirs principaux de traitement 7 et 14, de transfert C et C' au sein des premier et deuxième circuits de traitement 2 et 3, d'incubation D et D' et d'ajustement de pH E et E' dans les premier et deuxième réservoirs intermédiaires de traitement 9 et 16.

Les premier et deuxième réservoirs intermédiaires de traitement 9 et 16 peuvent être disposés sur le plateau 54, avec les première et deuxième unités de contrôle et de commande 61 et 62 qui se trouvent interposées entre les premier et deuxième réservoirs intermédiaires de traitement 9 et 16 et les premier et deuxième réservoirs principaux de traitement 7 et 14.

Le premier réservoir intermédiaire de traitement 9 peut se situer sur le premier côté du chariot 69 tandis que le deuxième réservoir intermédiaire de traitement 16 se situe sur le deuxième côté du chariot 69.

Les premier et deuxième réservoirs intermédiaires de traitement 9 et 16 peuvent tous deux être montés sur des balances respectives 57, comme les premier et deuxième réservoirs principaux de traitement 7 et 14, lesquelles sont pourvues de pieds, d'un plateau de balance 58 et d'un dispositif de contrôle et de commande de la balance 59. Les premier et deuxième réservoirs intermédiaires de traitement 9 et 16 peuvent être directement montés sur les plateaux de balance 58.

L'installation 1 peut comporter des dispositifs de transfert 64 et 65 disposée sur le plateau 54 à proximité respectivement des premier et deuxième réservoirs intermédiaire de traitement 9 et 16, et desquels saillent respectivement la première pompe intermédiaire d'alimentation 31 du premier côté du chariot 69 et la deuxième pompe intermédiaire d'alimentation 43 du deuxième côté du chariot 69.

Ces dispositifs de transfert 64 et 65 peuvent ainsi être configurés pour contrôler et commander les pompes susmentionnées pour mettre en oeuvre au moins les étapes de transfert F et F' du liquide biologique traité depuis les premier et deuxième circuits de traitement 2 et 3 vers le troisième réservoir principal de traitement 11 dans le circuit de sortie.

Le troisième réservoir principal de traitement 11 peut être interposé entre les premier et deuxième réservoirs intermédiaires de traitement 9 et 16.

Le troisième réservoir principal de traitement 11 peut être monté sur une balance 57, comme les premier et deuxième réservoirs principaux de traitement 7 et 14 et comme les premier et deuxième réservoirs intermédiaires de traitement 9 et 16, laquelle balance est pourvue de pieds, d'un plateau de balance 58 et d'un dispositif de contrôle et de commande de la balance 59. Le troisième réservoir principal de traitement 11 est directement monté sur le plateau de balance 58.

L'installation 1 peut comporter une troisième unité de contrôle et de commande 63 disposée sur le plateau 54 à proximité du troisième réservoir principal de traitement 11 et au niveau du troisième côté du chariot 69.

La troisième unité de contrôle et de commande 63 peut être pourvue d'un corps duquel saille partiellement la cinquième pompe acide 47 et la cinquième pompe base 49, du troisième côté du chariot 69.

La troisième unité de contrôle et de commande 63 peut ainsi être configurée pour contrôler et commander les pompes susmentionnées pour mettre en oeuvre au moins les étapes de pré-polissage dans le troisième réservoir principal de traitement 11.

L'installation 1 peut comporter des écrans de contrôle 66 montés sur un support fixé en saillie sur le plateau 54 du chariot 69. Ces écrans de contrôle 66 peuvent permettre de visualiser notamment le déroulement du procédé de traitement décrit plus haut et de gérer les paramètres associés à chacune des étapes de ce procédé.

Dans l'exemple décrit en référence aux figures 3 à 5, les premier, deuxième et troisième réservoirs principaux de traitement 7, 14 et 11 et les premier et deuxième réservoirs intermédiaires de traitement 9 et 16 peuvent être formés selon une même structure.

En particulier, ces réservoirs peuvent comporter une enceinte fermée 70 ici cylindrique et s'étendant entre un socle inférieur 72 et une plateforme supérieure 73, ainsi qu'un mécanisme d'agitation 71 pourvu d'un moteur saillant de la plateforme supérieure 73 et d'un agitateur (non visible) entraîné par le moteur et s'étendant dans l'enceinte 70.

Ces réservoirs peuvent en outre être pourvus d'un dispositif de filtration et/ou d'un évent 78 saillant de la plateforme supérieure 73 et d'organes d'instrumentation (non représentés) saillant également de la plateforme supérieure 73 et formés par exemple par un capteur de pression et par un capteur de température.

Ces réservoirs peuvent en outre être pourvus de plusieurs orifices d'entrée et/ou de sortie, situés au niveau du socle inférieur 72 et/ou au niveau de la plateforme supérieure 73 et/ou le long de l'enceinte 70. L'utilisation de ces différents orifices d'entrée et/ou de sortie dépend de la fonction des réservoirs.

En particulier, les premier et deuxième réservoirs principaux de traitement 7 et 14 peuvent être identiques et comporter chacun un premier orifice d'entrée 77 prévu pour être alimenté en liquide biologique et situé par exemple au bas de l'enceinte 70 près du socle inférieur 72, un deuxième orifice d'entrée 74 prévu pour être alimenté en acide et situé par exemple au bas de l'enceinte 70 près du socle inférieur 72, un troisième orifice d'entrée 75 prévu pour être alimenté en base et situé par exemple aussi au bas de l'enceinte 70 près du socle inférieur 72, et un orifice de sortie 76 prévu pour le transfert du liquide biologique vers le premier réservoir intermédiaire de traitement 9, respectivement le deuxième réservoir intermédiaire 16, et situé par exemple ici dans le socle inférieur 72.

Les premier et deuxième réservoirs intermédiaires de traitement 9 et 16 peuvent être identiques et similaires aux premier et deuxième réservoirs principaux de traitement 7 et 14, à l'exception d'un premier orifice d'entrée 80 qui est ici prévu pour être alimenté en liquide biologique depuis un réservoir principal respectif et qui par exemple saille de la plateforme supérieure 73, et d'un orifice de sortie 79 qui est prévu pour le transfert du liquide biologique vers le troisième réservoir principal de traitement 11 et qui est situé par exemple dans le socle inférieur 72.

Le troisième réservoir principal de traitement 11 peut être très similaire aux premier et deuxième réservoirs intermédiaires de traitement 9 et 16, si ce n'est que son premier orifice d'entrée 80 peut être prévu pour être alimenté en liquide biologique traité depuis un des premier et deuxième réservoirs intermédiaires de traitement 9 et 16, qu'il peut comporter un quatrième orifice d'entrée 81 prévu pour être alimenté en liquide biologique traité depuis l'autre des premier et deuxième réservoirs intermédiaires de traitement 9 et 16 et qui saille de la plateforme supérieure 73, et que son orifice de sortie 79 peut être prévu pour le transfert du liquide biologique la sortie de l'installation.

Dans des variantes non illustrées :
- l'installation peut être dépourvue de réservoirs intermédiaires de traitement, l'incubation se faisant alors dans les premier et deuxième réservoirs principaux ;
- les étapes relatives au deuxième circuit de traitement peuvent être lancées avant la fin du remplissage du premier réservoir principal de traitement dans le premier circuit de traitement ;
- le débit de vidange du troisième réservoir principal de traitement peut varier ou être fixe, dès lors qu'un flux continu de liquide biologique traité est fourni en sortie de l'installation ;
- l'installation peut comporter plus que deux circuits en parallèle, par exemple trois, quatre ou plus ;
- l'installation peut comporter plusieurs réservoirs de sortie communs à au moins deux circuits de traitement ;
- l'installation peut être agencée sur plusieurs chariots de préférence accolés les uns aux autres ;
- les réservoirs principaux et intermédiaires peuvent être agencés différemment les uns des autres et par exemple être pourvus de plus ou moins d'organes d'instrumentation ;
- les réservoirs principaux et intermédiaires peuvent être posés directement sur le plateau du chariot ;
- l'installation peut comporter une seule unité de contrôle et de commande ou bien deux, ou encore plus de trois unités de contrôle et de commande ; et/ou
- l'installation peut comporter un seul écran de contrôle ou bien plus que deux écrans de contrôle.

On rappelle plus généralement que l'invention ne se limite pas aux exemples décrits et représentés.

## Revendications

1. Installation de traitement de liquide biologique par inactivation virale, **caractérisée en ce qu'**elle comporte :
une vanne principale d'alimentation (4) en liquide biologique à traiter, configurée pour être raccordée au moins à une source d'alimentation (5) en liquide biologique ;
un premier circuit de traitement (2) en aval de ladite vanne d'alimentation et pourvu d'un premier réservoir principal de traitement (7) configuré pour être alimenté par ladite vanne d'alimentation en liquide biologique à traiter ;
un deuxième circuit de traitement (3) en aval de ladite vanne d'alimentation et pourvu d'un deuxième réservoir principal de traitement (14) configuré pour être alimenté par ladite vanne d'alimentation en liquide biologique à traiter ;
ledit deuxième circuit de traitement étant parallèle audit premier circuit de traitement ; et
un troisième réservoir principal de traitement (11) disposé en sortie à la fois dudit premier circuit de traitement et dudit deuxième circuit de traitement et configuré pour être alimenté successivement par ledit premier réservoir principal de traitement et par ledit deuxième réservoir principal de traitement ;
ladite installation est configurée pour que dans chacun desdits premier, deuxième et troisième réservoirs principaux de traitement, un volume déterminé d'acide et un volume déterminé de base est introduit de sorte au moins à ajuster un pH dudit liquide biologique ; et ladite installation est aussi configurée pour réguler un débit de vidange dudit troisième réservoir principal de traitement de sorte à fournir un débit continu de liquide biologique traité en sortie de ladite installation.

2. Installation selon la revendication 1, **caractérisée en ce que** ledit premier circuit de traitement (2) est pourvu d'un premier réservoir intermédiaire de traitement (9) disposé entre lesdits premier et troisième réservoirs principaux de traitement (7, 11).

3. Installation selon la revendication 2, **caractérisée en ce qu'**elle est configurée pour qu'un volume déterminé d'acide et qu'un volume déterminé de base soient introduits dans ledit premier réservoir intermédiaire de traitement (9).

4. Installation selon la revendication 3, **caractérisée en ce qu'**elle est configurée pour que ledit liquide biologique traité dans ledit premier réservoir principal de traitement (7 soit transféré dans ledit premier réservoir intermédiaire de traitement (9) et incube un temps prédéterminé dans ce dernier, avant son transfert dans ledit troisième réservoir principal de traitement (11).

5. Installation selon l'une quelconque des revendications 1 à 4, **caractérisée en ce que** ledit deuxième circuit de traitement (3) est pourvu d'un deuxième réservoir intermédiaire de traitement (16) disposé entre lesdits deuxième et troisième réservoirs principaux de traitement (14, 11).

6. Installation selon la revendication 5, **caractérisée en ce qu'**elle est configurée pour qu'un volume déterminé d'acide et qu'un volume déterminé de base soient introduits dans ledit deuxième réservoir intermédiaire de traitement (16).

7. Installation selon la revendication 6, **caractérisée en ce qu'**elle est configurée pour que ledit liquide biologique traité dans ledit deuxième réservoir principal de traitement (14) soit transféré dans ledit deuxième réservoir intermédiaire de traitement (16) et incube un temps prédéterminé dans ce dernier, avant son transfert dans ledit troisième réservoir principal de traitement (11).

8. Installation selon l'une quelconque des revendications 1 à 7, **caractérisée en ce qu'**elle comporte une pluralité de pompes d'alimentation (26, 31, 38, 43) disposées sur lesdits premiers et deuxième circuits de traitement (2, 3), au moins entre ledit premier réservoir principal de traitement (7) et ledit troisième réservoir principal de traitement (11), voire entre ledit premier réservoir principal de traitement (7) et ledit premier réservoir intermédiaire de traitement (9) et/ou entre ledit premier réservoir intermédiaire de traitement (9) et ledit troisième réservoir principal de traitement (11) ; et au moins entre ledit deuxième réservoir principal de traitement (14) et ledit troisième réservoir principal de traitement (11), voire entre ledit deuxième réservoir principal de traitement (14) et ledit deuxième réservoir intermédiaire de traitement (16) et/ou entre ledit deuxième réservoir intermédiaire de traitement (16) et ledit troisième réservoir principal de traitement (11).

9. Installation selon l'une quelconque des revendications 1 à 8, **caractérisée en ce que** lesdits volumes déterminés d'acide, respectivement lesdits volumes déterminés de base, introduits au moins dans lesdits premier, deuxième et troisième réservoirs principaux de traitement (7, 14, 11), voire dans lesdits premier et deuxième réservoirs intermédiaires de traitement (9, 16), dépendent des propriétés physicochimiques du liquide biologique à traiter et peuvent être égaux ou différents.

10. Installation selon l'une quelconque des revendications 1 à 9, **caractérisée en ce qu'**au moins lesdits premier, deuxième et troisième réservoirs principaux de traitement (7, 14, 11), voire lesdits premier et deuxième réservoirs intermédiaires de traitement (9, 16), sont pourvus d'un agitateur (71) de liquide biologique et/ou d'un ou plusieurs organes instrumentation.

11. Installation selon l'une quelconque des revendications 1 à 10, **caractérisée en ce qu'**elle comporte une ou plusieurs unités de contrôle et de commande 61, 62, 63) configurées pour contrôler et commander ladite vanne d'alimentation (4) et/ou au moins un desdits premier, deuxième et troisième réservoirs principaux de traitement (7, 14, 11), voire pour contrôler et commander lesdits premier et deuxième réservoirs intermédiaires de traitement (9, 16).

12. Installation selon l'une quelconque des revendications 1 à 11, **caractérisée en ce qu'**elle comporte un unique chariot (69) sur lequel sont disposés au moins ladite vanne d'alimentation (4), lesdits premier, deuxième et troisième réservoirs principaux de traitement (7, 14, 11), voire lesdits premier et deuxième réservoirs intermédiaires de traitement (9, 16).

13. Installation selon l'une quelconque des revendications 1 à 12, **caractérisée en ce qu'**elle est pourvue de conduites jetables reliant différents éléments la composant, dont au moins ladite vanne d'alimentation (4), lesdits premier, deuxième et troisième réservoirs principaux de traitement (7, 14, 11), voire lesdits premier et deuxième réservoirs intermédiaires de traitement (9, 16).

14. Procédé de traitement de liquide biologique par inactivation virale, à l'aide d'une installation de traitement (1) selon l'une quelconque des revendications précédentes, ledit procédé comportant :
une étape de remplissage au moins partiel en liquide biologique à traiter d'un premier réservoir principal de traitement (7) d'un premier circuit de traitement (2) de ladite installation via une vanne principale d'alimentation (4) de ladite installation ;
une étape d'ajustement de pH dudit liquide biologique en introduisant un volume déterminé d'acide et un volume déterminé de base dans ledit premier réservoir principal de traitement (7) ;
une étape d'incubation dudit liquide biologique dans ledit premier circuit de traitement (2) pendant un temps prédéterminé et une étape d'ajustement de pH dudit liquide biologique en introduisant un volume déterminé d'acide et un volume déterminé de base dans ledit premier circuit de traitement (2) pour se rapprocher d'un pH cible ;
une étape de transfert dudit liquide biologie traité dans un troisième réservoir principal de traitement (11) de ladite installation disposé en sortie dudit premier circuit de traitement (2), et une étape de pré-polissage en introduisant un volume déterminé d'acide et un volume déterminé de base dans ledit troisième réservoir principal de traitement (11) pour obtenir un liquide biologique traité au pH cible ;
en même que les étapes de pré-polissage et/ou de transfert et/ou d'incubation et/ou d'ajustements de pH mises en oeuvre dans ledit premier circuit de traitement (2), une étape de remplissage au moins partiel en liquide biologique à traiter d'un deuxième réservoir principal de traitement (14) d'un deuxième circuit de traitement (3) de ladite installation via ladite vanne principale d'alimentation (4), ledit deuxième circuit de traitement (3) étant parallèle audit premier circuit de traitement (2) ;
une étape d'ajustement de pH dudit liquide biologique en introduisant un volume déterminé d'acide et un volume déterminé de base dans ledit deuxième réservoir principal de traitement (14) ;
une étape d'incubation dudit liquide biologique dans ledit deuxième circuit de traitement (3) pendant un temps prédéterminé et une étape d'ajustement de pH dudit liquide biologique en introduisant un volume déterminé d'acide et un volume déterminé de base dans ledit deuxième circuit de traitement (3) pour se rapprocher d'un pH cible ;
une étape de transfert dudit liquide biologie traité dans ledit troisième réservoir principal de traitement (11) disposé aussi en sortie dudit deuxième circuit de traitement (3), et une étape de pré-polissage en introduisant un volume déterminé d'acide et un volume déterminé de base dans ledit troisième réservoir principal de traitement (11) pour obtenir un liquide biologique traité au pH cible ;
en même que les étapes de pré-polissage et/ou de transfert et/ou d'incubation et/ou d'ajustements de pH mises en oeuvre dans ledit deuxième circuit de traitement (3), une étape de vidange dudit troisième réservoir principal de traitement (11) en régulant un débit de vidange de sorte à fournir un débit continu de liquide biologique traité en sortie de ladite installation (1).

15. Procédé selon la revendications 14, **caractérisé en ce que** ledit premier circuit de traitement (2) est pourvu d'un premier réservoir intermédiaire de traitement (9) disposé entre lesdits premier et troisième réservoirs principaux de traitement (7, 11) et ledit deuxième circuit de traitement (3) est pourvu d'un deuxième réservoir intermédiaire de traitement (16) disposé entre lesdits deuxième et troisième réservoirs principaux de traitement (14, 11); et le procédé comporte une étape de transfert dudit liquide biologique depuis ledit premier réservoir principal de traitement (7) vers ledit premier réservoir intermédiaire de traitement (9) et les étapes d'incubation et d'ajustement de pH pour s'approcher d'un pH cible dans ledit premier circuit de traitement (2) ont lieu dans ledit premier réservoir intermédiaire de traitement (9) ; ainsi qu'une étape de transfert dudit liquide biologique depuis ledit deuxième réservoir principal de traitement (14) vers ledit deuxième réservoir intermédiaire de traitement (16) et les étapes d'incubation et d'ajustement de pH pour s'approcher d'un pH cible dans ledit deuxième circuit de traitement (3) ont lieu dans ledit deuxième réservoir intermédiaire de traitement (16).
